## Europäisches Patentamt

## European Patent Office

## Office européen des brevets

(11) Veröffentlichungsnummer: **0 094 614**
**A1**

(12) # EUROPÄISCHE PATENTANMELDUNG

(21) Anmeldenummer: **83104648.7**

(22) Anmeldetag: **11.05.83**

(51) Int. Cl.³: **A 61 F 5/44**

(30) Priorität: **14.05.82 DE 3218321**
**16.08.82 DE 3230447**

(43) Veröffentlichungstag der Anmeldung:
**23.11.83 Patentblatt 83/47**

(84) Benannte Vertragsstaaten:
**AT BE CH DE FR GB IT LI LU NL SE**

(71) Anmelder: **Hunger, Gerd, Dr.med.**
**Höslstrasse 8**
**D-8000 München 81(DE)**

(72) Erfinder: **Hunger, Gerd, Dr.med.**
**Höslstrasse 8**
**D-8000 München 81(DE)**

(74) Vertreter: **Vossius Vossius Tauchner Heunemann Rauh**
**Siebertstrasse 4 P.O. Box 86 07 67**
**D-8000 München 86(DE)**

(54) **Vorrichtung zum Auffangen des Harns bei Harninkontinenz des Mannes.**

(57) Gegenstand der Erfindung ist eine Vorrichtung zum Auffangen des Harns bei Harninkontinenz des Mannes mit einer am Unterleib des Mannes zu tragenden Halteeinrichtung und einer Einrichtung zum Auffangen des Harns. Die Halteeinrichtung ist als Skrotalgurt mit oder ohne Durchtrittsöffnung für den Penis oder als Unterhose oder Slip mit einer Durchtrittsöffnung für den Penis ausgebildet.

Die Einrichtung zum Auffangen des Harns ist - insbesondere bei Dranginkontinenz - ein wasserundurchlässiger Beutel oder - im Fall von Harnträufeln - ein Beutel aus luftdurchlässigem saugfähigem Material.

Statt der Einrichtung zum Auffangen des Harns kann auch eine Einrichtung zum Abklemmen der Harnröhre vorgesehen sein. Die Halteeinrichtung und die Einrichtung zum Auffangen des Harns bzw. die Einrichtung zum Abklemmen der Harnröhre können einstückig gearbeitet oder lösbar miteinander verbunden sein.

EP 0 094 614 A1

Croydon Printing Company Ltd.

VOSSIUS · VOSSIUS · TAUCHNER
HEUNEMANN · RAUH
PATENTANWÄLTE
SIEBERTSTR. 4, 8000 MÜNCHEN 80
TEL. (089) 47 40 75

0094614

u.Z.: S 401 EP          11. Mai 1983

Dr. G. Hunger

München, Bundesrepublik Deutschland

" Vorrichtung zum Auffangen des Harns bei Harninkontinenz

des Mannes "

Gemessen am Volumen gibt es zwei Grade der Harninkontinenz, des unwillkürlichen, schmerzlosen Harnabgangs:

1. Kleine Volumina - bei Störungen des Schließmuskels der Blase.

   a) Harnträufeln mit ständigem Abgang kleiner Harnmengen;

   b) Streßinkontinenz mit stoßweisem Abgang kleiner bis mäßiger Harnmengen, z.B. beim Husten, Heben oder beim Sport.

2. Große Volumina Hier handelt es sich um Dranginkontinenz (englisch urge incontinence), eine reflektorische Entleerung der gefüllten Harnblase. Im Strahl werden dabei Harnmengen in der Größenordnung von 250 ml entleert.

Die in der Erfindung vorgeschlagenen Prinzipien ermöglichen es, beide Formen besser als bisher zu behandeln.

Bisher wurden verwendet:

1) Windeln Sie lassen sich schlecht positionieren. Die Relation zwischen saugfähigem Material und aufgenommener Urinmenge ist ungünstig. Die Windeln sind voluminös und führen - wie vom Säugling allgemein bekannt - häufig zu Hautreizungen am Genital und an den Oberschenkeln.

2) Windelhöschen Das saugfähige Material ist im allgemeinen besser positioniert, aber keineswegs präzis in der Gegend der Harnröhrenöffnung. Vor allem lassen sich Windelhöschen nur sehr unbequem wechseln,und zwar beim amublanten wie beim bettlägerigen Träger.

3) Blasendauerkatheter Als Maßnahme zur Vereinfachung der Pflege inkontinenter Patienten muß er strengstens abgelehnt werden, weil er bei der weit überwiegenden Anzahl der Träger schon nach wenigen Tagen zu einer Infektion der Harnwege führt (Katheterfieber) und bei allen Trägern schon nach kurzer Liegezeit eine irreparable Schrumpfblase bedingt. Blasendauerkatheter dürfen heute nur auf ärztliche Anordnung und nur für wenige Tage verwendet werden.

4) Urinale Beim Kondom-Urinal ist die Abdichtung unzuverlässig – auch wenn zahlreiche Größen vorhanden sind. Fester Sitz wird vom Träger als störend empfunden, lockerer Sitz bringt die Gefahr des Abgleitens des Urinals bei der Dranginkontinenz mit sich. Unter dem Kondom-Urinal kommt es durch die dort ständig herrschende feuchte Atmosphäre leicht zu Hautreizungen und Hefebesiedelung.

5) Urinale mit aufblasbarer Manschette und Sicherheitsventil gegen Überdruck wurden entwickelt,

a) um das Urinal nur in einer oder nur in wenigen Größen herstellen zu müssen, weil mit der aufblasbaren Manschette individuelle Größendifferenzen überbrückt werden können;

b) zur Dauerfixierung des Urinals am Penis und zur gleichzeitigen wasserfesten Abdichtung; vgl. US-PSen 3 511 241, 3 916 902 und 4 013 077. Da die Dauerfixierung beim aktiven, nicht bettlägerigen Träger aber einen deutlich höheren Manschettendruck erfordert als die bloße Abdichtung gegen den geringen hydrostatischen Druck im Urinal, besteht wegen Beeinträchtigung der Blutzirkulation die Gefahr schwerer Gewebsschädigungen, wenn bei nicht bettlägerigen Trägern ausschließlich der Manschettendruck zur Dauerfixierung benutzt wird.

Leibgürtel mit recht komplizierten Halterungen wurden deshalb vorgeschlagen (vgl. US-PS 3 353 538), um die Dauerfixierung zu übernehmen, wobei dann der Manschettendruck nur so niedrig eingestellt werden muß, daß er gegen den geringen hydrostatischen Druck abdichtet.

Trotz dieser durchaus zweckmäßigen Trennung zwischen Fixierungs- und Abdichtungsdruck der Manschette blieb die Gefahr schwerer Gewebeschäden bei einem gelegentlichen Versagen des Sicherheitsventils bestehen. Deshalb und auch wohl aus Kostengründen haben auch die Urinale mit aufblasbarer Manschette kaum praktische Bedeutung gewonnen.

Wie kritisch Experten zu den bisherigen Lösungen stehen, zeigen Beurteilungen aus urologischen Standardwerken.

Bei Gummi-Urinalen, die direkt am Penis haften oder durch einen Hüftgurt gehalten werden, wird auf die Gefahr von Ulzera oder gar von Fisteln am Penis hingewiesen; vgl. Auerbach und Mitarb., "Handbuch der Urologie", Bd. XII, Springer Verlag, 1960, S. 1007. Die Notwendigkeit einer äußerst sorgfältigen Hautpflege wird betont.

Viele Querschnittsgelähmte benötigen wegen der Harninkontinenz ständig ein Urinal. Nach D.R. Smith, "Allgemeine Urologie", Urban und Schwarzenberg, 1968, S. 276, hat sich bisher noch keine einfache, zufriedenstellende Lösung dieser Probleme gefunden.

Gemäß Boeminghaus, "Urologie", Werkverlag Benaschewski, 1960, Bd. II, S. 586, ist das Urinal von allen Möglichkeiten die schlechteste Lösung, wobei mit operativem Vorgehen bei bestimmten Formen der Inkontinenz verglichen wird. Die

Penisklemme und eine aufblasbare Penis-Manschette werden - unter Hinweis auf ihre Gefahren - als Alternativen erwähnt.

Noch negativer wird der Katheter angesehen und festgestellt, daß jede Art der Katheterbehandlung der Inkontinenz unweigerlich zu einer Infektion der Harnwege führe; vgl. Boeminghaus, a.a.O., S. 369. Beim Querschnittsgelähmten kann sich daraus eine tödliche Urosepsis entwickeln.

In der US-PS 3 405 714 ist ein Einwegurinal beschrieben, das aus einem starren, rohrförmigen Kragen oder Verbindungsglied, einem inneren Einwegbeutel und einem äußeren oder Urinsammelbeutel besteht. Diese Teile sind mit einer Mehrzahl von Gurten am Körper des Trägers befestigt. Der starre Kragen weist an seinem oberen Ende einen Flansch mit Schlitzen auf, durch den die Gurte geführt werden. An seinem zylindrischen Teil befinden sich zwei Rillen, die der Befestigung der beiden Beutel dienen. Der innere Beutel endet in einem Lippenventil, das ein Zurückfließen des Urins aus dem äußeren Beutel verhindern soll. Der äußere Beutel wird mit Hilfe einer ringförmigen Klammer am Kragen wasserdicht befestigt.

Diese bekannte Vorrichtung besitzt eine ganze Reihe von Nachteilen. Da der Kragen starr ist, ist eine Abdichtung zwischen Penis und Kragen nicht möglich. Der Urin wird deshalb nur durch das Lippenventil des inneren Beutels vom Körper ferngehalten. Da - insbesondere beim Schlafen in Rückenlage - der Urin aus dem inneren Beutel nicht immer vollständig abfließt, befindet sich die betreffende Körperregion dauernd in feuchter Atmosphäre.

Die Befestigung des starren Kragens mit Gurten bedingt, daß sich dieser beim Sitzen und Bücken verschiebt und bei jeder Bewegung gegen den Körper drückt. Ein beschwerdefreies Tragen der Vorrichtung ist - insbesondere im Hinblick auf den

0094614

hervorstehenden Flansch - nicht möglich.

Schließlich ist das Wechseln der Teile nach dem Urinabgang besonders umständlich. Es müssen nämlich zwei Beutel abgenommen und ausgetauscht werden. Ferner muß der Kragen von drei Gurten gelöst und gereinigt werden.

Der Erfindung liegt die Aufgabe zugrunde, eine Vorrichtung zum Auffangen des Harns bei Harninkontinenz des Mannes zu schaffen, die mit einfachsten Mitteln jederzeit exakt in der gewünschten Position gehalten wird, die den Träger so wenig wie möglich behindert und bei der eine Dauerabdichtung zwischen Teilen der Vorrichtung und dem Penis nicht erforderlich ist. Diese Aufgabe wird durch die Erfindung gelöst.

Gegenstand der Erfindung ist somit eine Vorrichtung zum Auffangen des Harns bei Harninkontinenz des Mannes mit einer am Unterleib des Mannes zu tragenden Halteeinrichtung und einer Einrichtung zum Auffangen des Harns, die dadurch gekennzeichnet ist, daß die Halteeinrichtung als Skrotalgurt 1 mit einer Durchtrittsöffnung 2 für den Penis ausgebildet ist.

Gegenstand der Erfindung ist in einer anderen Ausführungsform eine Vorrichtung zum Auffangen des Harns bei Harninkontinenz des Mannes mit einer am Unterleib des Mannes zu tragenden Halteeinrichtung und einer Einrichtung zum Auffangen des Harns, dadurch gekennzeichnet, daß die Halteeinrichtung als Skrotalgurt (16) ohne Durchtrittsöffnung für den Penis ausgebildet ist.
Die Einrichtung zum Auffangen des Harns kann bei dieser Ausführungsform einen zylindrischen Hals (13) mit einer Aussparung (14) entsprechend der Peniswurzel aufweisen.

Gegenstand der Erfindung ist ferner in einer weiteren Ausführungsform eine Vorrichtung zum Auffangen des Harns bei Harninkontinenz des Mannes mit einer am Unterleib des Mannes zu tragenden Halteeinrichtung und einer Einrichtung zum Auffangen des Harns, dadurch gekennzeichnet, daß die Halteeinrichtung als Unterhose oder Slip (11) mit einer Durchtrittsöffnung (13) für den Penis ausgebildet ist.

Bei den drei vorstehend bezeichneten Ausführungsformen der Erfindung ist die Einrichtung zum Auffangen des Harns vorzugsweise ein wasserundurchlässiger Beutel mit einem zylindrischen Hals, und es ist eine Einrichtung zur Dauer- oder temporären ad hoc-Abdichtung des Bereiches zwischen dem Hals des Beutels und dem Penis vorgesehen, die dank der exakten Positionierung durch den Skrotalgurt bei beginnendem Harndrang sofort zu bewirken ist. Insbesondere kann die Einrichtung zur Abdichtung eine aufblasbare Manschette mit einem Schlauchansatz sein, wobei die mechanische Halterung des Auffangbeutels ausschließlich durch den Skrotalgurt oder den Slip geschieht, so daß die Abdichtung durch die Menschette mit einem geringen Druck erfolgen kann, der nur über dem hydrostatischen Druck des gefüllten Auffangbeutels zu liegen braucht, die Blutzirkulation aber nicht beeinträchtigt.

[insbesondere für die Dranginkontinenz]

In einer bevorzugten Ausführungsform ist die Innenseite des Halses des Beutels und der Manschette mit einer saugfähigen Beschichtung versehen.

[insbesondere für das Harnträufeln]

In einer anderen bevorzugten Ausführungsform ist die Einrichtung zum Auffangen des Harns ein Beutel aus luftdurchlässigem, saugfähigem Material. Der untere Teil des Beutels weist dann vorzugsweise eine Verstärkung aus wasserdichtem Material auf. Zur Erhöhung der Saugfähigkeit können ferner Einlagen aus saugfähigem Material in dem Beutel vorgesehen sein. Schließlich kann der Beutel aus einer dünnen, elastischen wasserdichten Folie bestehen und Einlagen aus saugfähigem Material sowie im oberen Teil Perforationen zur Herstellung der Luftdurchlässigkeit aufweisen.

Gegenstand der Erfindung ist schließlich in einer weiteren Ausführungsform eine Vorrichtung zum Auffangen des Harns bei Harninkontinenz des Mannes mit einer am Unterleib des Mannes zu tragenden Halteeinrichtung, dadurch gekennzeichnet, daß die Halteeinrichtung als Skrotalgurt mit oder ohne Durchtrittsöffnung oder als Unterhose oder Slip mit einer Durchtrittsöffnung für den Penis ausgebildet und eine damit verbundene Einrichtung zum Abklemmen der Harnröhre vorgesehen ist.

Die Einrichtung zum Abklemmen der Harnröhre ist vorzugsweise eine aufblasbare Manschette mit verstärkter Außenseite und dünner Innenseite oder eine einfache nicht elastische Manschette, die an ihrem in situ unteren Bereich eine Öffnung aufweist, durch welche ein im Inneren der Manschette zu einem aufblasbaren Körper erweiterten Schlauch führt.

Die Halteeinrichtung und die Einrichtung zum Auffangen des Harns bzw. die Einrichtung zum Abklemmen der Harnröhre sind in einer bevorzugten Ausführungsform der Erfindung einstückig gearbeitet bzw. unlösbar miteinander verbunden.

In einer anderen, ebenfalls bevorzugten Ausführungsform sind die Halteeinrichtung und die Einrichtung zum Auffangen des Harns bzw. die Einrichtung zum Abklemmen der Harnröhre lösbar miteinander verbunden. Die lösbare Verbindung zwischen der Halteeinrichtung und der Einrichtung zum Auffangen des Harns bzw. der Einrichtung zum Abklemmen der Harnröhre kann durch einen Klettenverschluß bewirkt werden. Der männliche Teil des Klettenverschlusses kann dabei ringförmig um die Durchtrittsöffnung des Skrotalgurts bzw. des Slips für den Penis und der weibliche Teil entsprechend ringförmig an der Einrichtung zum Auffangen des Harns bzw. der Einrichtung zum Abklemmen der Harnröhre angeordnet sein. Der weibliche Teil des Klettenverschlusses kann auch ein aus gewirktem Textil bestehender Ring sein. Ferner kann eine Lippe zum Abziehen der Einrichtung zum Auffangen des Harns bzw. der Einrichtung zum Abklemmen der Harnröhre vorgesehen sein.

Bei der Ausführungsform der Erfindung, bei der die Halteeinrichtung als Skrotalgurt ohne Durchtrittsöffnung ausgebildet ist, weist der zylindrische Hals der Auffangvorrichtung in einer bevorzugten Ausführungsform zwei Längsschlitze als Führung für den Skrotalgurt auf, die geschlossen sind oder vorzugsweise oben zu öffnen und zu verschließen sind. Zwischen dem zylindrischen Hals der Auffangvorrichtung und dem Skrotalgurt kann auch eine Knöpfverbindung bestehen.

Wenn die Halteeinrichtung als Slip ausgebildet ist, dann weist das Material zwischen einem elastischen Gürtelband und der Durchtrittsöffnung des Slips vorzugsweise eine unelastische Verstärkung auf.

Die Erfindung wird nachstehend mit Bezug auf die Zeichnung näher erläutert. Es zeigen:

Fig. 1    eine Ausführungsform des erfindungsgemäß verwendeten Skrotalgurts mit Durchtrittsöffnung für den Penis,

       Fig. 1a: Skrotalgurt, Seitenansicht
       Fig. 1b: Skrotalgurt, Vorderansicht

Fig. 2    eine Ausführungsform der Erfindung, in der der Skrotalgurt mit einem wasserundurchlässigen Beutel zusammen mit einer aufblasbaren Manschette vorgesehen ist;

       Fig. 2a:  Auffangbeutel und Manschette, Seitenansicht
       Fig. 2b:  Auffangbeutel und Manschette, Vorderansicht.

Fig. 3    eine Ausführungsform der Erfindung, in der der Skrotalgurt mit einem Auffangbeutel aus luftdurchlässigem, saugfähigem Material vorgesehen ist.

Fig. 3a:   Auffangbeutel, Seitenansicht
Fig. 3b:   Auffangbeutel, Verschlußseite.

Fig. 4a    - eine Seitenansicht bzw. eine Rückansicht des Ober-
und b        teils einer Ausführungsform der erfindungsgemäßen Vorrichtung mit Skrotalgurt ohne Durchtrittsöffnung;

Fig. 5a    - eine Seitenansicht bzw. eine Vorderansicht des Ober-
und b        teils einer anderen Ausführungsform der erfindungsgemäßen Vorrichtung mit Skrotalgurt ohne Durchtrittsöffnung;

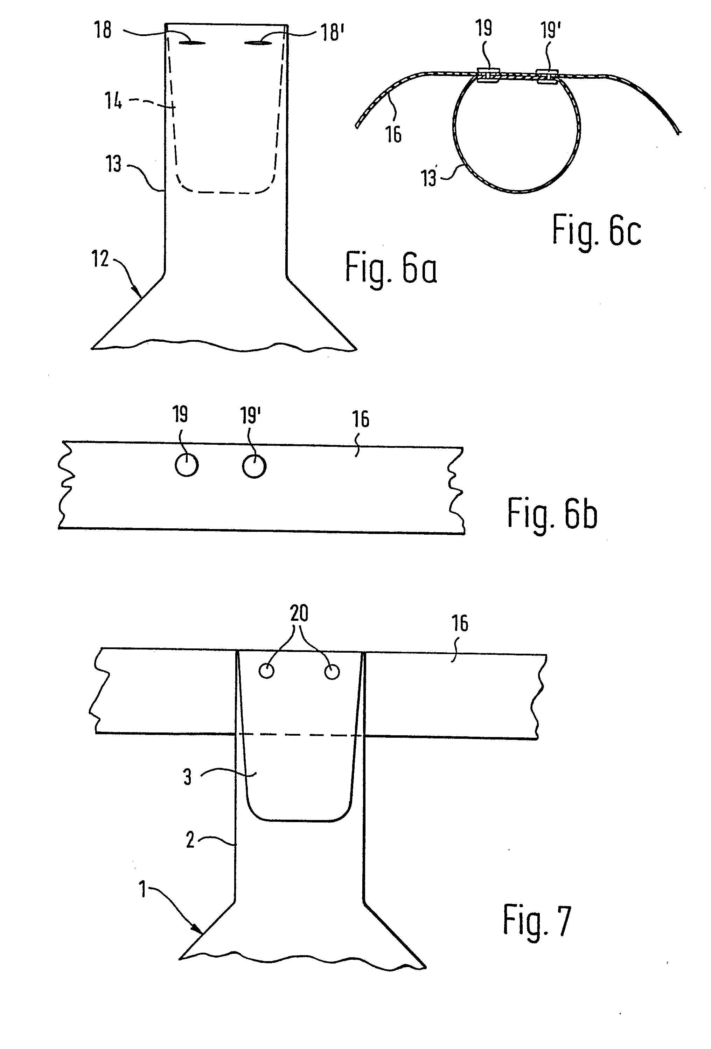

Fig. 6a    - eine weitere Ausführungsform der erfindungsgemäßen
bis c        Vorrichtung (Skrotalgurt ohne Durchtrittsöffnung) mit einer Vorderansicht des Halses des Auffangbehälters (Fig. 6a), einer Aufsicht eines Teils des Skrotalgurtes (Fig. 6b) und einen Querschnitt durch die zusammengesetzte Auffangvorrichtung im Bereich der Knöpfverbindung zwischen dem Skrotalgurt und dem Auffangbehälter (Fig 6c);

Fig. 7    - eine Rückansicht einer weiteren Ausführungsform der erfindungsgemäßen Vorrichtung (Skrotalgurt ohne Durchtrittsöffnung) mit flächiger Fixierung des Skrotalgurtes am Hals des Auffangbehälters;

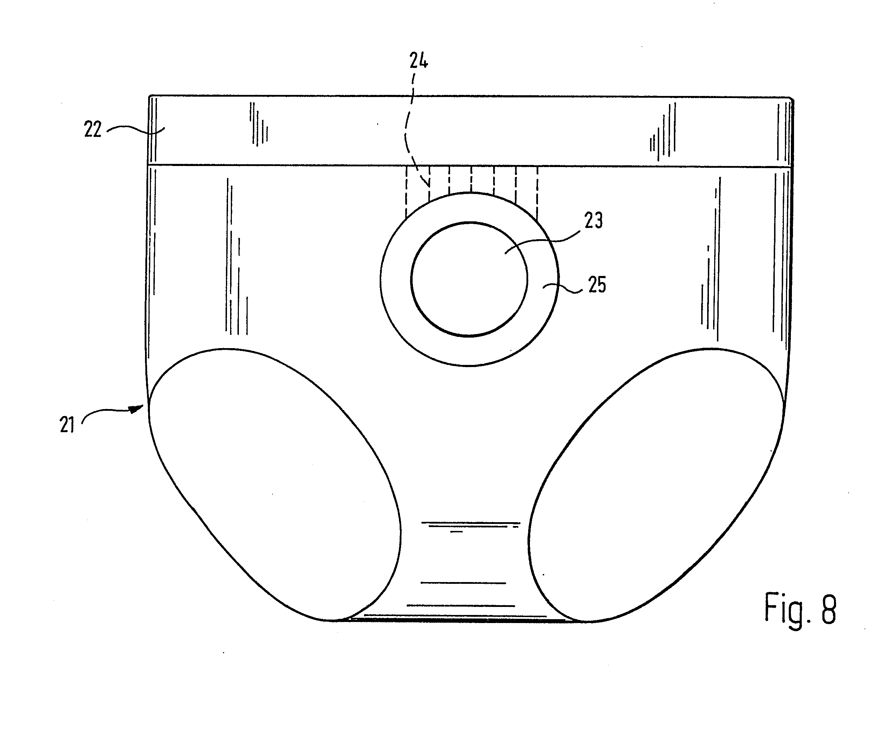

Fig. 8    - eine Vorderansicht einer Ausführungsform der Halteeinrichtung in Form eines Slip mit Durchtrittsöffnung;

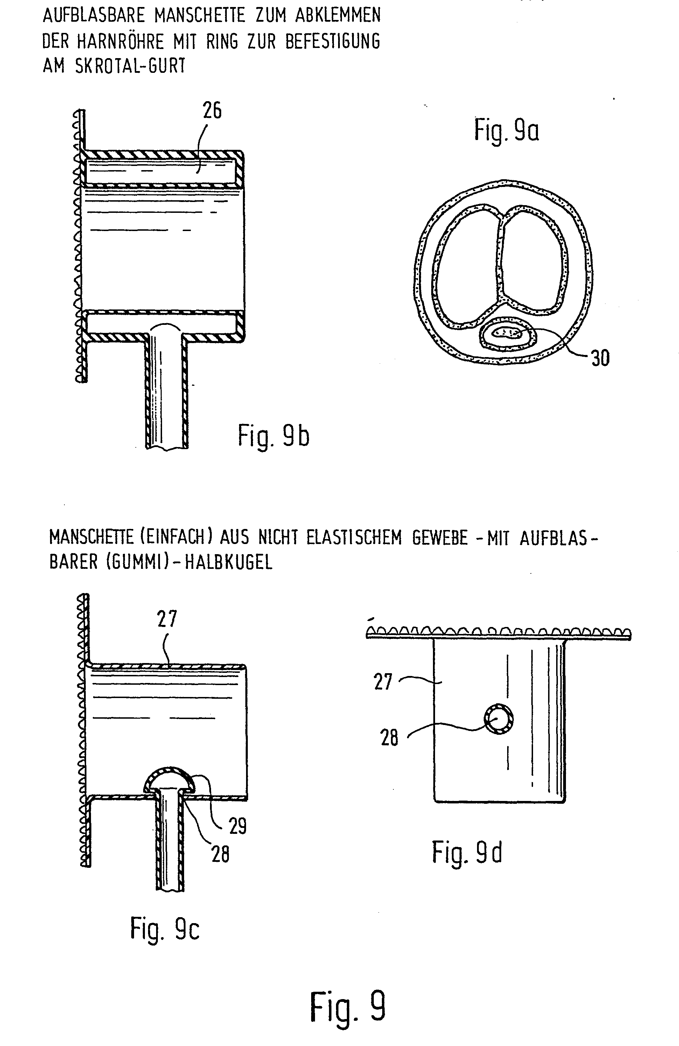

Fig. 9      eine Ausführungsform der Erfindung, in der zusammen mit einem Skrotalgurt eine Einrichtung zum
            Abklemmen der Harnröhre vorgesehen ist;

Fig. 9a:    Querschnitt durch den Penis mit der
            exzentrisch angeordneten Harnröhre
Fig. 9b:    aufblasbare Manschette mit verstärkter
            Außenwand
Fig. 9c:    einfache nicht elastische Manschette mit
            aufblasbarem Gummikörper
Fig. 9d:    Vorrichtung gemäß Fig. 9c; Ansicht
            der Manschette von unten.

Die Auffang- oder Abklemmvorrichtung der Erfindung stellt in mehrfacher
Hinsicht einen Fortschritt dar. Sie wird durch einen Skrotalgurt mit oder
ohne Durchtrittsöffnung - also mit einem einfachen und preiswerten Mittel -
jederzeit exakt in der gewünschten Position gehalten. Das Gleiche gilt für
die Ausführungsform, bei der die Halteeinrichtung als Unterhose oder Slip ausgebildet ist. Bei Verwendung einer aufblasbaren Manschette zur Abdichtung des Bereiches zwischen dem
Penis und dem Hals des Harnauffangbeutels erübrigt sich eine
Dauerabdichtung, da die Manschette dank der exakten Positionierung durch den Skrotalgurt bzw. die Unterhose nur kurzfristig,
nämlich vom Beginn des Harndrangs bis zum Wechseln des Beutels,
aufgeblasen sein muß. Auch im Fall der Verwendung eines
Beutels aus luftdurchlässigen, saugfähigem Material ist dank
der exakten Positionierung ein Abdichten am Penis nicht erforderlich. Ähnliches gilt für die Ausführungsform der Vorrichtung, bei der eine Einrichtung zum Abklemmen der Harnröhre
vorgesehen ist. Auch hier muß die Manschette dank der Positionierung durch einen Skrotalgurt oder die Unterhose nur kurzfristig - nämlich zwischen Harndrang und alsbaldiger Entleerung der Blase - aufgeblasen werden.

Durch die Vorrichtung der Erfindung wird die gar nicht zu unterschätzende Gefahr von Gewebsschädigungen durch Überdruck ausgeschaltet, und zwar zuverlässiger und preiswerter als durch Sicherheitsventile. Der Leibgurt mit seinen komplizierten Halterungen wird durch einen einfachen Skrotalgurt oder Slip ersetzt. Dabei wird eine Dauerabdichtung am Penis durch eine kurzzeitige ad hoc-Abdichtung ersetzt. Die in verschiedenen Ausführungsformen der Erfindung verwendete aufblasbare Manschette kann sowohl zur Abdichtung am Penis als auch zur Abdichtung des Auffangbeutels dienen.

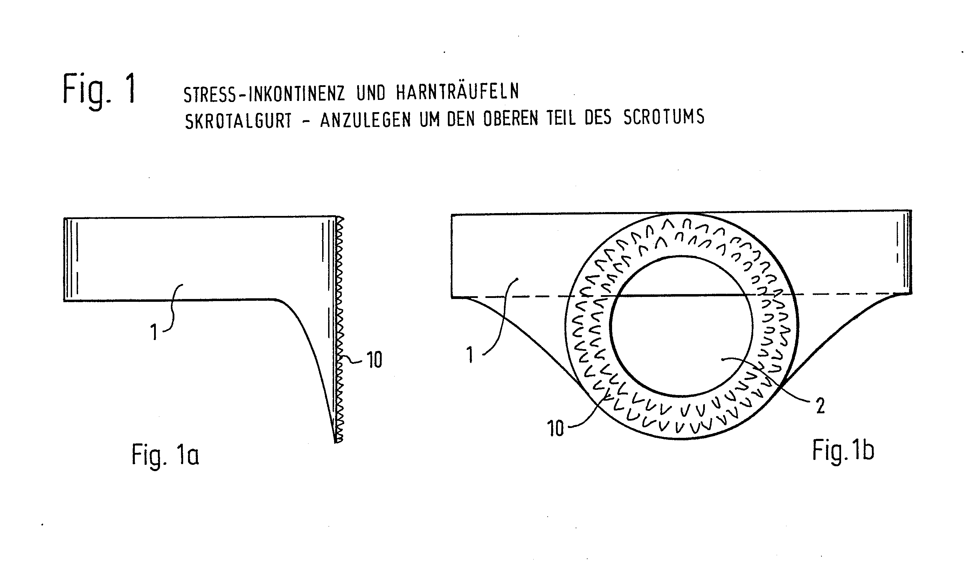

Nachstehend wird die Ausführungsform der Vorrichtung der Erfindung näher erläutert, die in den Fig. 1 bis 3 dargestellt ist. Sie eignet sich für aktive, nicht bettlägerige Patienten, kann aber natürlich auch von inaktiven, bettlägerigen Patienten benutzt werden. Die Vorrichtung besteht aus einem wiederverwendbaren oder als Einwegartikel gestalteten Skrotalgurt, der rund um den oberen verengten Teil des wegen seines Aufbaues aus Muskel- und Faszienschichten durchaus belastbaren Scrotkums gelegt wird, und der für den Durchtritt des Penis eine kreisförmige Öffnung 2 hat (Fig. 1).

Falls Halteeinrichtung und Einrichtung zum Auffangen des Harns voneinander lösbar ausgestaltet sind, ist diese Öffnung von einem Teil eines Verschlusses 10 umgeben, dessen Gegenstück 11 sich am Einmal-Auffangbeutel befindet. Die Art des Verschlusses ist an sich unerheblich, doch eignen sich wegen der Behaarung dieser Re- gion Klebe-, Schnapp- oder auch einrastende Verschlüsse weniger, weil Haare verklebt oder eingeklemmt werden können. Günstig ist der männliche Teil eines Klettenverschlusses, weil beim Befestigen des Auffangbeutels eingefangene Haare ohne Belästigung herausgleiten können. Auch Knöpfe oder Steckschlaufen entsprechen dieser Anforderung. Die zugehörigen Einmal-Beutel haben vorzugsweise einen entsprechenden Ring mit dem weiblichen Teil eines Klettenverschlusses - oder einfacher, billiger und auch leichter zu trennen, einen aus gewirkten Textilien bestehenden Ring oder Knopflöcher.

Erfindungsgemäß können die Halteeinrichtung und die Einrichtung zum Auffangen des Harns jedoch auch unlösbar miteinander verbunden ·sein. Beispielsweise kann der Skrotalgurt mit einem Beutel als Einrichtung zum Auffangen des Harns in einer der nachstehend erläuterten Ausführungen einstückig gearbeitet sein. Diese unlösbar verbundene Ausführungsform erspart die Kosten für den Verschluß zwischen Skrotalgurt und Beutel und ist wegen ihrer Wirtschaftlichkeit bevorzugt.

Nun garantiert der Scrotalgurt zwar eine ganz präzise Positionierung der Harnröhrenöffnung im Inneren des Beutels, doch scheint vor allem bei der für die Querschnittsgelähmten charakteristischen Dranginkontinenz ein wasser- und damit praktisch luftdichter Abschluß zwischen Penis und Auffangbeutel unvermeidbar zu sein. Da aber bei fast allen diesen Patienten doch einige Sekunden zwischen dem Auftreten des Harndrangs und der Harnentleerung im Strahl vergehen (vgl. "Ärztliche Praxis XXXIV, Nr. 22 (1982), S. 843), konnte durch die Kombination des Scrotalgurts mit dem Prinzip der aufblasbaren Manschette auch für die am schwierigsten zu behandelnde Dranginkontinenz eine ganz neuartige Lösung gefunden werden. Sie vermeidet bei vielen dieser Patienten den dauernden schädlichen Kontakt der Penishaut mit einem wasserdichten, dampfdichten und praktisch luftdichten Material.

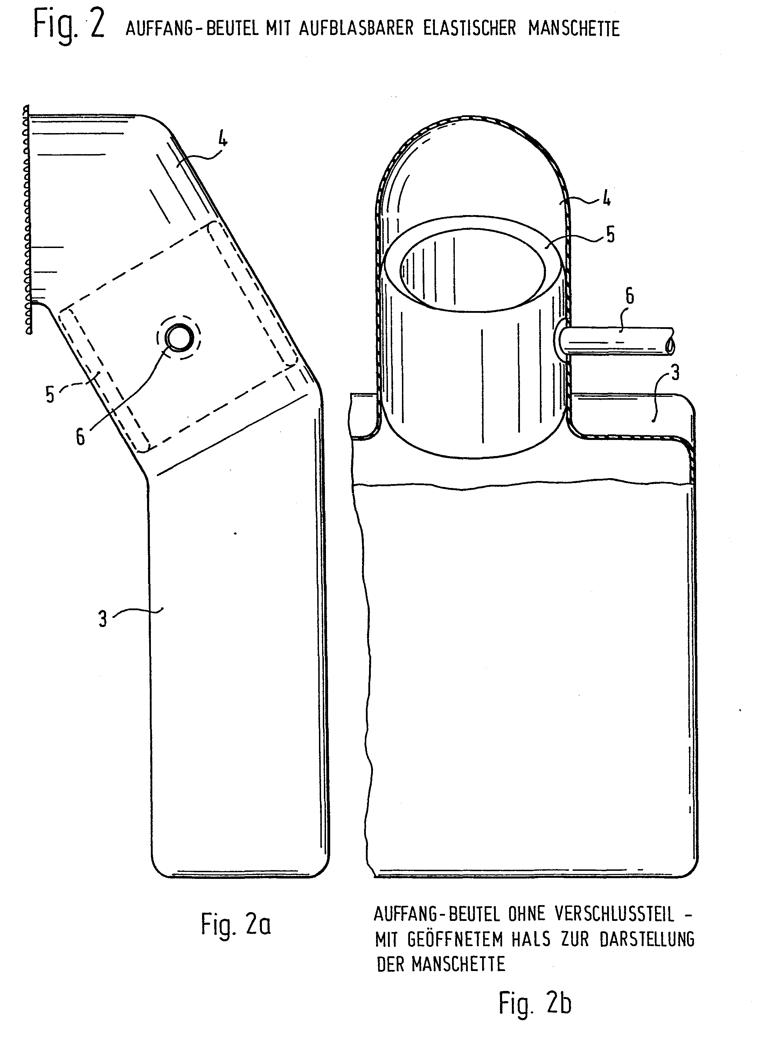

Bei Dranginkontinenz ist der Hals 4 des Auffangbeutels 3 mit einer aufblasbaren, elastischen Manschette 5 ausgerüstet. Der Beutel (Fig. 2) ist wasserundurchlässig und hat ein Fassungsvermögen in der Größenordnung von mindestens 250 ml. Er hat einen zylindrischen Hals, mit einem schräg zur Halsachse stehenden Ring ausgestattet, auf dem der weibliche Teil eines Klettenverschlusses oder das Gegenstück eines anderen Verschlusses aufgebracht ist. In dem zylindrischen Hals ist eine aufblasbare Gummimanschette befestigt mit einem Schlauch 6, der durch ein Loch im Hals des Beutels oder auch zwischen den beiden Verschlußteilen (nicht gezeichnet) nach außen führt.

Bei dieser Ausführungsform der Erfindung ist weder ein wasserdichter Abschluß zwischen dem Schlauch und dem Hals des Beutels, noch zwischen Außenseite der Manschette und Hals des Beutels erforderlich. Das bedeutet eine Einsparung bei der Produktion: Die wasserfeste Abdichtung erfolgt beim Aufblasen (vgl. Fig. 2). Zur Vereinfachung der Produktion kann sogar auf jede feste Verbindung zwischen Beutelhals und Manschette verzichtet werden, weil beim Aufblasen ein doppelter Zweck erreicht wird: Innen Abdichtung rund um den Penis, außen Abdichtung des Beutelhalses und damit eine feste Verbindung dieser beiden Teile der Vorrichtung.

Beim Anlegen wird bei dieser Ausführungsform zunächst die Manschette übergestreift, dann ihr Schlauch von innen nach außen durch die Öffnung im Hals geführt und dann der Beutel übergestreift. Der Schlauch kann auch zwischen den beiden Teilen des Verschlusses nach außen geführt werden. Die Manschette und der Ansatz des Beutels sind absichtlich weit gehalten; erstens gibt es dann keine Schwierigkeiten bei dem Überstreifen bei individuellen Benutzern; zweitens bleibt eine ausreichende Luftzirkulation erhalten, abgesehen von der kurzen Zeit, in der die Gummimanschette aufgeblasen ist. Die Befestigung an dem Scrotalgurt garantiert, daß der Auffangbeutel stets präzise seine gewünschte Position beibehält. Deshalb kann auf eine Dauerabdichtung verzichtet werden. Erst bei beginnendem Drang wird in Sekundenschnelle die Manschette aufgeblasen und das ganze System wasserdicht gemacht. Dazu wird eine wiederverwendbare Ballonpumpe mit einer Möglichkeit zum Ablassen des Druckes benutzt, wie sie bei der Blutdruckmessung üblich ist. Diese Pumpe kann unauffällig in der Hosentasche getragen werden. Ihr ausreichend langer Schlauch wird durch ein kleines Loch in der Wand der Hosentasche zu dem Schlauchansatz der aufblasbaren Manschette geführt. Die Pumpe bleibt abgesehen vom Wechseln der Beutel immer mit der Manschette verbunden. Beutel werden umgehend nach jeder Harnentleerung gewechselt.

Eine weitere Verfeinerung des Systems besteht in einer saugfähigen Beschichtung der Innenseite des Beutelhalses sowie
der Gummimanschette.

Einzelne wenige Patienten empfinden aber keinerlei Vorwarnzeit vor der Harnentleerung im Strahl. Auch bei diesen
bringt der Scrotalgurt Vorteile. Er ist simpel und sicher
und wesentlich bequemer zu tragen und einfacher anzulegen
als ein Leibgurt mit Halterungen, er erlaubt aber ebenfalls
die Trennung der beiden Teilfunktionen.

1. Fixierung bei Zugbelastung durch den gefüllten Beutel
   - garantiert durch die mechanische Verbindung mit dem Skrotalgurt
   und

2. Abdichtung - garantiert durch die aufblasbare, elastische
   Manschette.

Da der Skrotalgurt die mechanische Fixierung übernimmt, kann man
bei der Abdichtung mit einem sehr geringen Manschettendruck
auskommen, der nur über dem hydrostatischen Druck des gefüllten Auffangbeutels zu liegen braucht und deshalb die Gefahr
von Stauungserscheinungen bei der Blutzirkulation ausschließt.

Ein Rückschlagventil im Beutel sowie ein Anschluß für
einen Schlauch, der zu einem Sammelbehälter führt, können
in einer weiteren Ausführungsform vorgesehen sein. Sie sind
überflüssig, wenn gefüllte Beutel umgehend gewechselt werden.

Der Scrotalgurt kann beim Harnträufeln oder bei der Streßinkontinenz, also bei kleinen Harnvolumina, aber auch dazu
dienen, die erforderlichen Auffangmaterialien bei ambulanten oder liegenden Patienten ganz präzise in der gewünschten Position, nämlich gegenüber der äußeren Harnröhrenöffnung, zu fixieren. Ein wasserdichter Abschluß am Penis wird
dadurch überflüssig. Das ist in doppelter Hinsicht vorteilhaft: Keine Beeinträchtigung der Blutversorgung, keine Beeinträchtigung der Penishaut.

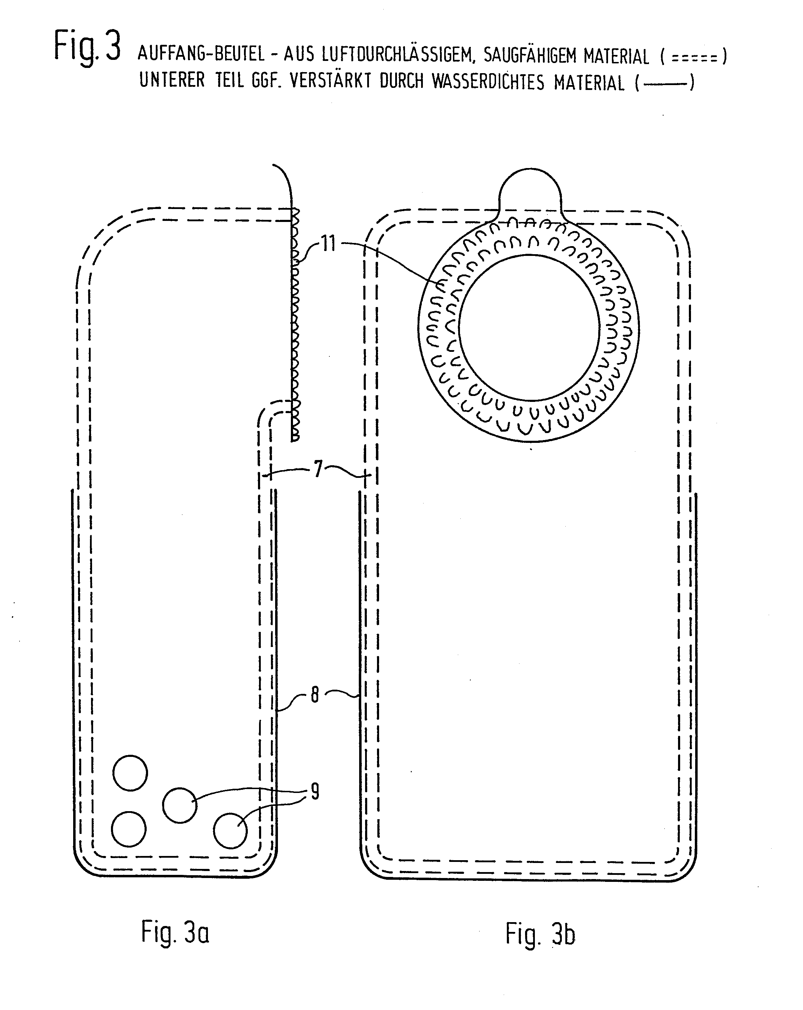

1. Geringstes Harnvolumen Hier ist ein Auffangbeutel 7 ausschließlich aus saugfähigem, "atmendem" Material (Zellstoffwatte, Zellstofflaum, Spezialmaterial mit hoher Saugfähigkeit) ausreichend. Kein Abschluß durch luftdichtes Material belastet die Penishaut; vgl. Fig. 3. Die Form des Beutels ist unerheblich. Er kann z.B. auch die in Fig. 2 gezeigte Form haben.

2. Geringes Harnvolumen Die Saugfähigkeit kann durch Einlegen von passend geformtem, saugfähigem Material 9, z.B. Tampons, in den Beutel verstärkt werden.

3. Kleines Harnvolumen

a) Der saugfähige Beutel erhält an dem - in situ - unteren Teil einen wasserdichten Überzug 8. Der obere Teil und der Hals bleiben luftdurchlässig.

b) Die Saugfähigkeit kann auch hier noch zusätzlich durch Einlegen passend geformten Materials erhöht werden.

4. Es besteht auch die Möglichkeit, den Beutel preisgünstig aus dünner, elastischer, wasserdichter, evtl. geprägter Folie (wegen der Geräuscharmut) herzustellen, wobei dann erstens die Einlage von passend geformtem, saugfähigem Material die Absorption der Feuchtigkeit sicherstellt und zweitens der obere Teil durch feine Perforationen luftdurchlässig gemacht wird.

Bei der beschriebenen Ausführungsform der Erfindung handelt es sich also um eine Vorrichtung bei den unterschiedlichen Formen der Harninkontinenz des Mannes, bestehend aus einem Skrotalgurt und verschiedenartigen Auffangbeuteln. Der Skrotalgurt umschließt den oberen Teil des Scrotums und weist für den Penis eine Durchtrittsöffnung auf, die im Fall einer lösbaren Verbindung von dem einen Teil eines Verschlusses umgeben ist. Das Gegenstück des Verschlusses befindet sich am Auffangbeutel. Dieser Verschluß braucht nicht wasserdicht zu sein. DerAuffangbeutel kann auch unlösbar mit dem Skrotalgurt verbunden sein bzw. einstückig mit ihm gearbeitet sein.

Bei kleinem Harnvolumen (Harnträufeln, Streßinkontinenz) liegt der entscheidende Vorteil gegenüber den üblichen Windeln oder Windelhöschen in der ganz präzisen Positionierung der Auffangmaterialien an der richtigen Stelle, nämlich der äußeren Harnröhrenöffnung, unabhängig von körperlicher Aktivität oder unruhigem Schlaf. Diese durch den Scrotalgurt bedingte präzise Positionierung unter allen Eventualitäten erlaubt es, mit einem Minimum an saugfähigem Material einen maximalen Effekt zu erzielen, und erlaubt es außerdem, in allen Inkontinenzfällen mit geringem Harnvolumen auf einen wasser- und damit luftdichten Abschluß zu verzichten, wodurch Hautirritationen und Hefeinfektionen eliminiert werden. Die Hautverträglichkeit von saugfähigem Material ist generell besser als diejenige von wasserundurchlässigem Material. Bei Bedarf kann die Aufnahmekapazität des Beutels durch Einlegen von passend geformtem, saugfähigem Material, z.B. Tampons, individuell vergrößert werden, wobei wiederum eine exakte Positionierung maximale Aufsaugung mit minimalen Materialmengen ermöglicht. Gegenüber Windeln und Windelhöschen lassen sich die vorgeschlagenen Auffangbeutel auch wesentlich leichter auswechseln und bewahren vor allem Skrotum und Damm vor einer ständigen Benetzung mit Urin.

Bei großen Harnvolumina (Dranginkontinenz) sind wasserdichte Beutel unvermeidlich. Die Frontseite des Skrotalgurts kann hier zu einer Art Schürze verlängert werden, um den unangenehmen Kontakt zwischen Skrotalhaut und Folienbeutel zu vermeiden. In Verbindung mit dem Skrotalgurt läßt sich eine Dauerabdichtung zum Penis hin vermeiden. Dazu werden Auffangbeutel mit weitem Hals und weiter Manschette verwendet. Dies erlaubt einerseits die so erwünschte Luftzirkulation, andererseits werden die Beutel durch den Scrotalgurt stets präzis positioniert, so daß die bei den allermeisten Patienten vorhandene Vorwarnzeit genügt, um die dünnwandige Luftmanschette aufzublasen und so in Sekunden zwischen Penis und Beutel einen wasserdichten Abschluß herzustellen. Diese Abdichtung bleibt aber nur ganz kurzfristig bestehen, denn der Auffangbeutel wird nach jeder Harnentleerung umgehend gewechselt. Auffangbeutel und Manschette brauchen bei der Produktion nicht verbunden zu werden, was Kosteneinsparungen erlaubt. Durch das Aufblasen wird ein doppelter Effekt erreicht: Innen Abdichtung rund um den Penis, außen Abdichtung des Auffangbeutels – und damit Verbindung dieser zwei Teile der Vorrichtung.

Bei den wenigen Patienten ohne jede Vorwarnzeit liegt der Vorteil des Scrotalgurts darin, daß die Trennung von a) Fixierung des Beutels gegenüber dem Zug des gefüllten Beutels und b) Abschluß zwischen Auffangbeutel und Penis gegenüber dem hydrostatischen Druck des gefüllten Beutels mit einfacheren Mitteln als bisher erfolgt. Diese Trennung der Funktionen erlaubt es, die Abdichtung mit minimalem Druck vorzunehmen, wodurch vor allem in der Langzeitbehandlung Stauungserscheinungen vermieden werden. Bei diesen Patienten kann die Erfindung vielfach den Blasendauerkatheter mit seinen schweren gesundheitlichen Folgen ersetzen.

Die vorstehenden Ausführungen gelten in entsprechender Weise für die Ausführungsform der Erfindung gemäß Fig. 8, bei der die Halteeinrichtung statt als Skrotalgurt als Unterhose oder Slip mit einer Durchtrittsöffnung für den Penis ausgebildet ist.

Diese Ausführungsform ist besonders vorteilhaft für ältere Menschen, die sich nicht umgewöhnen wollen oder können. Ein weiterer Vorteil ist darin zu sehen, daß die psychologische Belastung, ein sanitäres Hilfsmittel tragen zu müssen durch eine ganz normal aussehende Unterhose, wesentlich geringer wird.

Bevorzugt weist das Hosenmaterial, das z.B. ein Gewebe sein kann, zwischen dem elastischen Gürtelband und der Durchtrittsöffnung eine unelastische Verstärkung auf, um ein Verrutschen der Vorrichtung, insbesondere bei gefülltem Auffangbehälter, zu verhindern.

Die Unterhose weist rund um die Durchtrittsöffnung den Teil des Verschlusses auf, wie er sich in der vorstehend erläuterten Ausführungsform am Skrotalgurt befindet. Alle Ausführungen des Auffangbeutels gemäß vorstehender Erläuterung können auch in Kombination mit der Unterhose bzw. dem Slip verwendet werden.

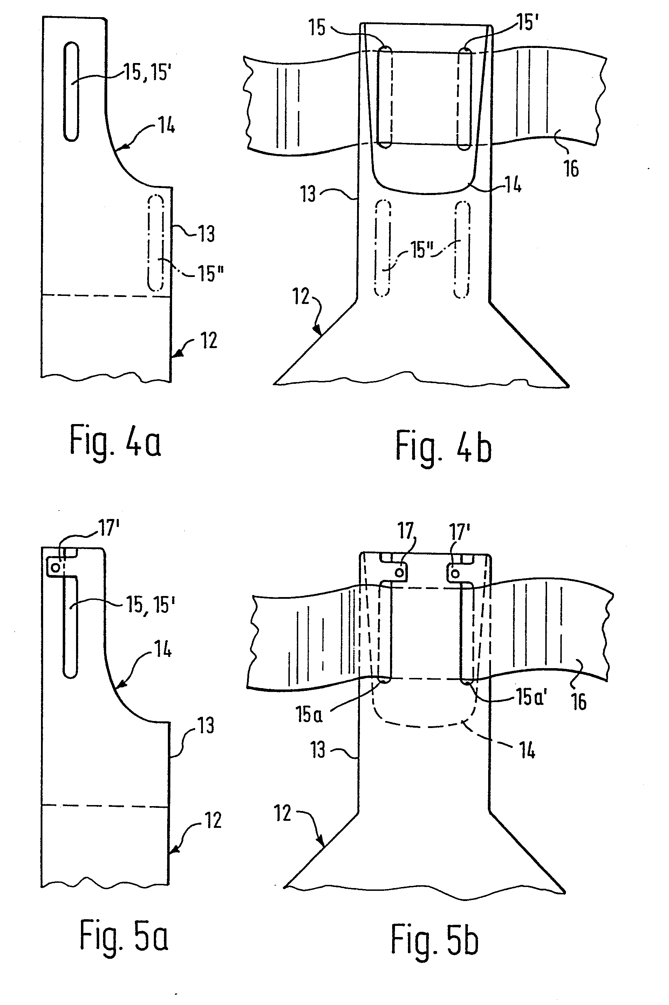

In einer weiteren, in den Fig. 4 bis 7 dargestellten Ausführungsform weist der Skrotalgurt keine Durchtrittsöffnung für den Penis auf; zweckmäßig wird der zylindrische Hals der Auffangvorrichtung mit einer Aussparung versehen, durch die die Auffangvorrichtung an die Form der Peniswurzel angepaßt wird. Durch diese Maßnahme ist es möglich, mit Hilfe eines relativ einfachen und preiswerten Skrotalgurtes den Auffangbehälter jederzeit exakt in der gewünschten Position zu halten.

Eine besonders einfache Verbindung zwischen dem Skrotalgurt und dem Auffangbehälter erhält man durch zwei Längsschlitze im zylindrischen Hals des Auffangbehälters, durch die der Skrotalgurt gezogen wird und so als Führung für letzteren dienen. Dadurch ist es möglich, den Auffangbehälter längs dem Skrotalgurt zu verschieben, ohne diesen abzunehmen.

Bei einer bevorzugten Ausgestaltung sind die Längsschlitze im Hals des Auffangbehälters oben offen und können mit Hilfe eines Verschlusses dort geschlossen werden, so daß etwa beim Wechseln eines Auffangbehälters lediglich die Verschlußeinrichtung an den Schlitzen geöffnet werden muß, um den Auffangbehälter vom Skrotalgurt abzuziehen, der dabei am Körper des Patienten im gegurteten Zustand verbleibt; entsprechend kann ein neuer Auffangbehälter wieder mit dem Skrotalgurt verbunden werden.

Als Verschlußeinrichtung kann eine Knöpfverbindung oder ein Klettenverschluß oder eine Verknotung vorgesehen sein, um den Auffangbehälter in einfacher Weise vom Skrotalgurt zu lösen bzw. mit diesem zu verbinden. Zu diesem Zweck sind beispielsweise am Skrotalgurt Knöpfe und im Hals der Auffangvorrichtung Knopflöcher vorgesehen; eine umgekehrte Anordnung ist ebenfalls möglich.

Erfindungsgemäß kann auch der Skrotalgurt fest mit dem Hals der Auffangvorrichtung verbunden werden, beispielsweise durch flächiges Fixieren (z.B. Verkleben) des Halses am Skrotalgurt. Dies vereinfacht das Anlegen der Auffangvorrichtung einschließlich Skrotalgurt, die dann als Wegwerfartikel eingesetzt werden.

Diese erfindungsgemäßen Ausführungsformen vereinfachen die Vorrichtungen für die Verbindung zwischen Skrotalgurt und Auffangvorrichtung oder sparen diese Verbindungsvorrichtungen gänzlich ein, so daß eine besonders kostengünstige Herstellung möglich ist.

Bei den nachstehend im Zusammenhang mit den Figuren 4 bis 7 dargestellten Ausführungsformen wird der Skrotalgurt oberhalb der Peniswurzel angelegt, d. h. der Skrotalgurt befindet sich an der dem Körper des Patienten abgewandten Seite des Auffangbehälterhalses.Ein Anlegen des Skrotalgurtes unterhalb der Peniswurzel ist im Rahmen der Erfindung ebenfalls möglich; der Auffangbehälter befindet sich dann an der Außenseite des Skrotalgurtes und die Aussparung bzw. die Position des Skrotalgurtes am Behälterhals ist entsprechend zu modifizieren.

Im Rahmen der Erfingung können auch bei den vorstehend erläuterten Ausführungsformen jeweils eine Abdichteinrichtung vorgesehen sein, wie sie vorstehend im Zusammenhang mit der Ausführungsform gemäß Fig. 1 bis 3 erläutert ist.

Die in den Figuren 4 bis 7 dargestellten Ausführungsformen, weisen einige wesentliche gemeinsame Merkmale auf. In diesen Fällen ist für die Aufnahme des Harns ein nur teilweise dargestellter Auffangbehälter oder -beutel 12 mit oben angesetztem zylindrischem Hals 13 vorgesehen. An der dem Körper des Patienten zugewandten Seite am oberen Ende des zylindrischen Halses ist eine Aussparung 14 ausgebildet, deren Form die der Peniswurzel angepaßt ist, um ein möglichst paßgerechtes und beschwerdefreies Einlegen des Penis in den Hals zu ermöglichen.

Zum Haltern des Auffangbehälters 12 am Körper des Patienten ist in allen vier dargestellten Fällen ein Skrotalgurt 16 ohne Durchtrittsöffnung für den Penis vorgesehen.

Unterschiede zwischen den Ausführungsformen der Fig. 4 bis 7 bestehen im wesentlichen in der Verbindung zwischen dem Skrotalgurt 16 und dem Hals 13 des Auffangbehälters 12. So ist bei der Ausführungsform gemäß Fig. 4 der zylindrsiche Hals 13 am oberen Ende etwa in Höhe der Aussparung 14 mit zwei im wesentlichen zueinander parallelen Schlitzen 15, 15' versehen, die sich etwa in Längsrichtung des zylindrischen Halses 13 erstrecken. Zum Haltern des Auffangbehälters 12 mit Hilfe des mehrfach verwendbaren Skrotalgurtes 16 wird letzterer durch die Schlitze 15, 15' geführt, wie dies in Figur 4b angedeutet ist. Bei dieser Ausführung der Schlitze 15, 15' muß zum Auswechseln des Auffangbehälters 12 der Skrotalgurt 16 vom Körper des Patienten angenommen werden.

Demgegenüber bietet die Ausführungsform gemäß Fig. 5 eine Erleichterung beim Wechseln des Auffangbehälters 13, denn hier sind die Schlitze 15, 15' an sich oben offen und können mit Hilfe von beispielsweise knöpfbaren Lappen 17, 17' ⌐oder auch mit einem Knoten⌐ am oberen Ende des Halses 13 verschlossen werden. Soll bei dieser Ausführungsform ein Auffangbehälter 12 ausgewechselt werden, so werden die Schlitze 15a, 15a' durch Lösen der Lappen 17, 17' ⌐oder der Knoten⌐ am oberen Ende geöffnet, so daß bei weiter am Körper des Patienten verbleibenden Skrotalgurt 16 der Auffangbehälter 12 nach unten herausgezogen werden kann. Entsprechend wird umgekehrt ein neuer Auffangbehälter mit zunächst oben offenen Schlitzen 15a, 15a' von unten auf den Skrotalgurt 16 aufgesteckt, und anschließend werden die Schlitze 15a, 15a' am oberen Ende mit Hilfe der Lappen 17, 17' ⌐oder mit Knoten⌐ verschlossen, wie dies in Fig. 5b dargestellt ist.

Als Vorteilhaft bei den Ausführungsformen der Figuren 4 und 5 ist die Möglichkeit der Verschiebung des Auffangbehälters 12 entlang des Skrotalgurtes anzusehen, um so bei bereits angelegtem Skrotalgurt 16 eine optimale Positionierung des Auffangbehälters zu ermöglichen.

Bei der in Fig. 6 dargestellten Ausführungsform ist anstelle der Schlitze der Ausführungsformen gemäß Fig. 4 und 5 zwischen dem Hals 13 des Auffangbehälters 13 und dem Skrotalgurt 16 eine Knöpfverbindung vorgesehen, beispielsweise mit Knopflöchern 18, 18' im Bereich des oberen Endes des nicht ausgesparten Teils des Halses 13 sowie mit entsprechenden Knöpfen am Skrotalgurt. Selbstverständlich können im Rahmen der Erfindung auch die Knöpfe am Hals des Auffangbeutels und die Knopflöcher im Skrotalgurt angeordnet sein, Diese Ausführungsform ermöglicht ein sehr rasches und einfaches Austauschen des Auffangbehälters 12.

Bei der in Fig. 7 dargestellten Ausführungsform ist der Skrotalgurt 16 am oberen Ende des Halses 13 gegenüber der Aussparung 14 flächig fixiert, beispielsweise durch Verschweißen oder Verkleben an den Stellen 20. Ein derartiger, nur einmal verwendbarer Wegwerfartikel kann relativ preiswert hergestellt werden, und das Anlegen dieser einstückigen Vorrichtung ist relativ einfach.

In Abwandlung der vorstehenden Ausführungsformen der Figuren 4 bis 7 kann der Skrotalgurt auch auf der dem Körper des Patienten zugewandten Seite des Auffangbehälterhalses, z.B. knapp unterhalb der Aussparung 14 (in den Figuren 4a und 5a rechts) angeordnet sein und/oder selbst an der entsprechenden Stelle eine passende Aussparung für die Peniswurzel aufweisen (vgl. z.B. die strichpunktierten Führungsschlitze 15" in Fig. 4 anstelle der Schlitze 15, 15').

Die Ausführungsform der Erfindung , in der die Haltevorrichtung als Unterhose oder Slip mit einer Durchtrittsöffnung für den Penis ausgebildet ist, ist in Fig. 8 dargestellt. Am oberen Ende des Slip 21 befindet sic- in an sich bekannter Weise ein elastisches Gürtelband 22. Zwischen dem Gürtelband 22 und der Durchtrittsöffnung 23 kann das Slipmaterial, z.B. in Form eines Gewebes, mit einer unelastischen Verstärkung 24 versehen sein, um das Gewicht des an der Durchtrittsöffnung 23 zu befestigenden Auffangbehälters abzufangen.

Verschiedene Ausführungsformen eines zusammen mit dem Slip gemäß Fig. 8 verwendbaren Auffangbehälters sind in den Fig. 2 und 3 dargestellt. Die Durchtrittsöffnung 23 im Slip 21 ist von einem Teil eines Verschlusses 25 umgeben, dessen Gegenstück sich am Einmal-Auffangbeutel oder -behälter befindet.

Der Skrotalgurt mit oder ohne Durchtrittsöffnung bzw. der Slip mit Durchtrittsöffnung für den Penis bieten noch eine weitere Möglichkeit, die Drang-Inkontinenz zu beherrschen und zwar ohne jeden Auffangbeutel, womit auch eine Belastung der Penishaut praktisch entfällt. Bei dieser Ausführungsform, die in Fig. 9 dargestellt ist, wird eine aufblasbare Manschette 26 mit einer Außenwand aus nur mäßig elastischem Material (z.B. stärkerwandiger Gummi) am Skrotalgurt 1 oder am Slip 21 befestigt (Fig. 9b). Die Manschette ist vorzugsweise zylindrisch und hat eine Breite von etwa 2 cm. Mit Beginn des Dranges wird sie soweit aufgeblasen, daß der Preßdruck der Manschette höher ist als der Preßdruck der Blase. Da es dabei zu einer Behinderung von Blutrückfluß oder auch Blutzufluß kommt, müssen Druckentlastung und Entleerung der Blase umgehend eingeleitet werden.

Eine Modifikation dieses Prinzips erlaubt es, den notwendigen Druck zum Verschluß der Harnröhre gezielter und damit für die Blutversorgung schonender auszuüben. Die Harnröhre 30 liegt im Penisschaft nämlich exzentrisch; vgl. Fig. 9a. Deshalb hat eine am Skrotalgurt 1 oder am Slip 21 zu befestigende einfache Manschette 27 aus nicht elastischem Gewebe an der Unterseite ein Loch 28 durch das ein Schlauch zu einem innerhalb der Manschette liegenden, aufblasbaren Gummikörper 29, beispielsweise einer Gummi-Halbkugel, führt; vgl. Fig. 9c und 9d. Durch diese Anordnung ist der gezielte Druck auf die Harnröhre höher als auf die im Penisschaft verteilten Blutgefäße. Aber auch hier muß der Benutzer wissen, daß diese pneumatische Abklemmung wegen der unvermeidlichen Beeinträchtigung der Blutversorgung nur ganz kurzfristig erfolgen darf, daß er also unverzüglich durch Ablassen des Druckes die Blase entleeren muß.

Die in Fig. 9 gezeigten Ausführungsformen einer Manschette zur Abklemmung der Harnröhre können in einer weiteren Ausgestaltung auch fest (unlösbar) mit dem Skrotalgurt bzw. der Unterhose verbunden sein.

Das Ausführungsbeispiel erläutert die Erfindung.

### B e i s p i e l

Die Vorrichtung zum Auffangen des Harns bei Harninkontinenz wird in der Praxis in folgender Weise benutzt:

Zunächst wird der Scrotalgurt angelegt, und zwar so, daß er ohne Kompression den oberen Teil des Scrotums umschließt, andererseits aber auch nicht zu locker sitzt. Der Penis wird dabei durch die vom Verschluß umgebene kreisförmige Öffnung nach außen geführt.

Bei geringem Harnvolumen wird nun einfach ein Auffangbeutel aus saugfähigem Material mit seinem Verschlußteil am Gegenstück des am Scrotalgurt befindlichen Verschlusses befestigt. Feuchte Beutel werden ausgewechselt, was auch durch den Hosenschlitz erfolgen kann.

Bei großen Harnvolumina wird der Beutel, der beispielsweise aus PVC-Folie mit ausblasbarer Gummimanschette in seinem Hals besteht, in gleicher Weise am Scrotalgurt befestigt. Das Überstreifen über den Penis kann durch kreisförmige Verstärkungslinien am Hals des Beutels erleichtert werden. Sie erlauben ein Spreizen des Halses. In der Hosentasche befindet sich eine Ballonpumpe, wie sie beim Blutdruckmessen verwendet wird. Durch ein kleines Loch in der Hosentasche wird der Schlauch mit dem Schlauch der aufblasbaren Manschette verbunden. Die Manschette wird aber nicht aufgeblasen. Sie ist weit und erlaubt so den Zutritt von Luft. Erst bei beginnendem Harndrang wird die Abdichtung mit ein oder zwei Pumpbewegungen in Sekundenschnelle hergestellt. Der erforderliche Abdichtungsdruck liegt dabei unter dem Entleerungsdruck der Blase. Unmittelbar nach der Entleerung des Urins in den Beutel wird der Manschettendruck über das Auslaßventil an der Ballonpumpe abgelassen, die Verbindung der Schläuche gelöst und der Beutel gewechselt.

Sinngemäß gelten diese Ausführungen auch für den Skrotalgurt ohne Durchtrittsöffnung.

Bei pneumatischer Abklemmung der Harnröhre wird zunächst genauso vorgegangen. Hier ist wegen des höheren Druckes die umgehende Druckentlastung der Manschette mit Entleerung der Harnblase auf der Toilette oder in ein Uringlas besonders wichtig.

Bei Patienten ohne Vorwarnzeit muß die Manschette zwar ständig aufgepumpt sein, es wird aber nur ein ganz geringer Druck von einigen $cmH_2O$ benötigt, wodurch sich Störungen der Blutversorgung vermeiden oder minimieren lassen.

<u>Patentansprüche</u>

1. Vorrichtung zum Auffangen des Harns bei Harninkontinenz des Mannes mit einer am Unterleib des Mannes zu tragenden Halteeinrichtung und einer Einrichtung zum Auffangen des Harns, d a d u r c h  g e k e n n z e i c h n e t , daß die Halteeinrichtung als Skrotalgurt (1) mit einer Durchtrittsöffnung (2) für den Penis ausgebildet ist.

2. Vorrichtung zum Auffangen des Harns bei Harninkontinenz des Mannes mit einer am Unterleib des Mannes zu tragenden Halteeinrichtung, und einer Einrichtung zum Auffangen des Harns, dadurch gekennzeichnet, daß die Halteeinrichtung als Skrotalgurt (16) ohne Durchtrittsöffnung für den Penis ausgebildet ist.

3. Vorrichtung zum Auffangen des Harns bei Harninkontinenz des Mannes mit einer am Unterleib des Mannes zu tragenden Halteeinrichtung und einer Einrichtung zum Auffangen des Harnes, dadurch gekennzeichnet, daß die Halteeinrichtung als Unterhose oder Slip (21) mit einer Durchtrittsöffnung (23) für den Penis ausgebildet ist.

4. Vorrichtung nach Anspruch 1, 2 oder 3, dadurch gekennzeichnet, daß die Einrichtung zum Auffangen des Harns ein wasserundurchlässiger Beutel (3) mit einem zylindrischen Hals (4) ist, und daß eine Einrichtung zur Dauer- oder zur temporären ad hoc-Abdichtung des Bereiches zwischen dem Hals des Beutels und dem Penis vorgesehen ist, die dank der exakten Positionierung durch den Skrotalgurt bei beginnendem Harndrang sofort zu bewirken ist.

5. Vorrichtung nach Anspruch 4, dadurch gekennzeichnet, daß die Einrichtung zur Abdichtung eine aufblasbare Manschette (5) mit einem Schlauchansatz (6) ist, wobei die mechanische Halterung des Auffangbeutels ausschließlich durch den Skrotalgurt oder den Slip geschieht, so daß die Abdichtung durch die Manschette mit einem sehr geringen Druck erfolgen kann, der nur über dem hydrostatischen Druck des gefüllten Auffangbeutels zu liegen braucht, die Blutzirkulation aber nicht beeinträchtigt.

6.   Vorrichtung nach Anspruch 1 bis 3, dadurch gekennzeichnet, daß die Halteeinrichtung als Skrotalgurt mit oder ohne Durchtrittsöffnung oder als Unterhose oder Slip mit einer Durchtrittsöffnung für den Penis ausgebildet und eine damit verbundene Einrichtung zum Abklemmen der Harnröhre vorgesehen ist.

7.   Vorrichtung nach Anspruch 1, 2 und 4 bis 6, dadurch gekennzeichnet, daß die Halteeinrichtung und die Einrichtung zum Auffangen des Harns bzw. die Einrichtung zum Abklemmen der Harnröhre einstückig gearbeitet bzw. unlösbar miteinander verbunden sind.

8.   Vorrichtung nach Anspruch 1 bis 6, dadurch gekennzeichnet, daß die Halteeinrichtung und die Einrichtung zum Auffangen des Harns bzw. die Einrichtung zum Abklemmen der Harnröhre lösbar miteinander verbunden sind.

9.   Vorrichtung nach Anspruch 2, 4, 5 und 8, dadurch gekennzeichnet, daß die Einrichtung zum Auffangen des Harns einen zylindrischen Hals (13) mit einer Aussparung (14) entsprechend der Peniswurzel aufweist.

10.   Vorrichtung nach Anspruch 2, 4, 5, 8 und 9, dadurch gekennzeichnet, daß der zylindrische Hals (13) des Auffangbehälters (12) zwei Längsschlitze (15, 15', 15'') als Führung für den Skrotalgurt (16) aufweist, die geschlossen sind oder oben zu öffnen und zu verschließen sind.

# Fig. 1

STRESS-INKONTINENZ UND HARNTRÄUFELN

SKROTALGURT – ANZULEGEN UM DEN OBEREN TEIL DES SCROTUMS

Fig. 1a

Fig. 1b

Fig. 2  AUFFANG-BEUTEL MIT AUFBLASBARER ELASTISCHER MANSCHETTE

Fig. 2a

AUFFANG-BEUTEL OHNE VERSCHLUSSTEIL –
MIT GEÖFFNETEM HALS ZUR DARSTELLUNG
DER MANSCHETTE

Fig. 2b

Fig. 3 AUFFANG-BEUTEL – AUS LUFTDURCHLÄSSIGEM, SAUGFÄHIGEM MATERIAL ( ===== )
UNTERER TEIL GGF. VERSTÄRKT DURCH WASSERDICHTES MATERIAL ( ——— )

Fig. 3a

Fig. 3b

15, 15'

14

13

15"

12

## Fig. 4a

15

15'

14

16

13

15"

12

## Fig. 4b

17'

15, 15'

14

13

12

## Fig. 5a

17

17'

15a

15a'

16

13

14

12

## Fig. 5b

Fig. 6a

Fig. 6c

Fig. 6b

Fig. 7

Fig. 8

AUFBLASBARE MANSCHETTE ZUM ABKLEMMEN
DER HARNRÖHRE MIT RING ZUR BEFESTIGUNG
AM SKROTAL-GURT

Fig. 9a

Fig. 9b

MANSCHETTE (EINFACH) AUS NICHT ELASTISCHEM GEWEBE - MIT AUFBLAS-
BARER (GUMMI)-HALBKUGEL

Fig. 9c

Fig. 9d

Fig. 9

Europäisches
Patentamt

EUROPÄISCHER RECHERCHENBERICHT

**0094614**
Nummer der Anmeldung

EP 83 10 4648

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X,A | US-A-3 357 430 (ROSENBERG) * Spalte 3, Zeilen 60-67; Figur 8 * | 1,2,4, 6,8 | A 61 F 5/44 |
| X,A | US-A-3 356 091 (PATTERSON) * Figur 1 * | 3,4,6, 8 | |
| X,A | US-A-3 721 243 (HESTERMAN et al.) * Figur 1 * | 3,4,6, 8 | |
| A | GB-A-2 036 561 (GARDINER) * Figuren 1, 2 * | 6 | |
| A | US-A-2 455 859 (FOLEY) * Figuren 1, 4, 5 * | 6 | RECHERCHIERTE SACHGEBIETE (Int. Cl. ³) |
| A | US-A-2 640 484 (JOHNSON) | | A 61 F 5/00 |
| A | US-A-3 397 698 (HICKEY) | | |
| A | DE-C- 867 582 ("SANUSSA" PEDERSEN & CO.) | | |
| D,A | US-A-3 405 714 (MOSS) | | |

--- -/-

Der vorliegende Recherchenbericht wurde für alle Patentansprüche erstellt.

| Recherchenort BERLIN | Abschlußdatum der Recherche 05-08-1983 | Prüfer KANAL P K |
|---|---|---|

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| D,A | US-A-3 353 538 (CARRIGAN) | | |
| D,A | US-A-4 013 077 (RITOTA et al.) | | |
| D,A | US-A-3 916 902 (LINEBERGER) | | |
| D,A | US-A-3 511 241 (LEE) | | |

RECHERCHIERTE SACHGEBIETE (Int. Cl. ³)

Der vorliegende Recherchenbericht wurde für alle Patentansprüche erstellt.

| Recherchenort | Abschlußdatum der Recherche | Prüfer |
|---|---|---|
| BERLIN | 05-08-1983 | KANAL P K |